(19) Europäisches Patentamt
European Patent Office
Office européen des brevets

(11) Publication number: **0 115 201**
**B1**

(12) **EUROPEAN PATENT SPECIFICATION**

(45) Date of publication of patent specification: **02.03.88**

(51) Int. Cl.$^4$: **G 03 C 1/72,** C 07 D 495/10 // (C07D495/10, 335:00, 209:00)

(21) Application number: **83307960.1**

(22) Date of filing: **23.12.83**

(54) **Photosensitive compositions containing photochromic compounds.**

(30) Priority: **28.12.82 JP 233036/82**
**02.09.83 JP 161702/83**

(43) Date of publication of application:
**08.08.84 Bulletin 84/32**

(45) Publication of the grant of the patent:
**02.03.88 Bulletin 88/09**

(84) Designated Contracting States:
**DE FR GB NL**

(56) References cited:
**GB-A-1 168 965**

**CHEMICAL ABSTRACTS, Vol. 98, 1983, pages 566-567, abstract nr. 125243m, COLUMBUS, OHIO, (US) A.S. KHOLMANSKII et al.: "Orbital nature of phosphorescent states of spiropyrans with a nitro group".**

(73) Proprietor: **SONY CORPORATION**
**7-35 Kitashinagawa 6-Chome Shinagawa-ku**
**Tokyo 141 (JP)**

(72) Inventor: **Arakawa, Seiichi**
**c/o Sony Corporation 7-35 Kitashinagawa 6-chome**
**Shinagawa-ku Tokyo (JP)**
Inventor: **Kondo, Hirofumi**
**c/o Sony Corporation 7-35 Kitashinagawa 6-chome**
**Shinagawa-ku Tokyo (JP)**
Inventor: **Seto, Junetsu**
**c/o Sony Corporation 7-35 Kitashinagawa 6-chome**
**Shinagawa-ku Tokyo (JP)**

(74) Representative: **Cotter, Ivan John et al**
**D. YOUNG & CO. 10 Staple Inn**
**London WC1V 7RD (GB)**

**EP 0 115 201 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to photosensitive compositions and materials containing photochromic compounds, for use (for instance) in the optical recording art.

Photochromic photosensitive spiropyran materials are known to have a number of advantages: (1) high resolution can be obtained without involving any particulate property; (2) no specific developing and fixing treatments are needed; (3) thin film formation is possible because of high intensity of the formed colour; and (4) erasion and rewriting are possible. Accordingly, attempts have been made to apply these materials to various recording and memory materials and also to duplicating materials. Especially in recent years, application of these materials as recording media, e.g. optical video discs, in which a laser beam is used to record and reproduce information signals while making use of features (1) and (4) indicated above, has been expected to take place.

Known photochromic, photosensitive spiropyran materials have an absorption wavelength, in the coloured state, ranging at most from 400 nm to 700 nm. For laser beam recording or reproducing purposes, it is essential to use a gas laser such as an $Ar^+$ or He-Ne laser. As is well known, small-size, light-weight semiconductor lasers have recently been developed. So, there is a tendency for semiconductor lasers to take the place of gas lasers for recording and reproducing purposes.

Semiconductor lasers actually applied to laser recording or reproducing operations have emitting wavelengths ranging from 780 to 850 nm. At present, semiconductor lasers which emit at shorter wavelengths have been extensively developed. It is considered that there is a high possibility of putting into practice semiconductor lasers emitting at about 700 nm in the very near future (see, for example, "Present State and Future of Semiconductor Laser Recording", Journal of Japanese Photographic Association 44(2) (1981), by Fujitaro Saito, and "Development and Present State of Visible Light Semiconductor Lasers", Optronics (No. 9), 41 (1982)).

In order to apply photochromic photosensitive spiropyran materials as a recording medium for semiconductor laser recording and reproducing purposes, the materials should have good absorption characteristics with respect to light having a wavelength higher than used in the prior art cases and, in particular, a wavelength higher than 700 nm.

As is known in the art, the following spirothiopyran compound has a good absorption characteristic, in the coloured state, with respect to light having the range of wavelengths mentioned above (see J. Phys. Chem. 72, 997 (1981), by H. S. Becker and J. Kolc).

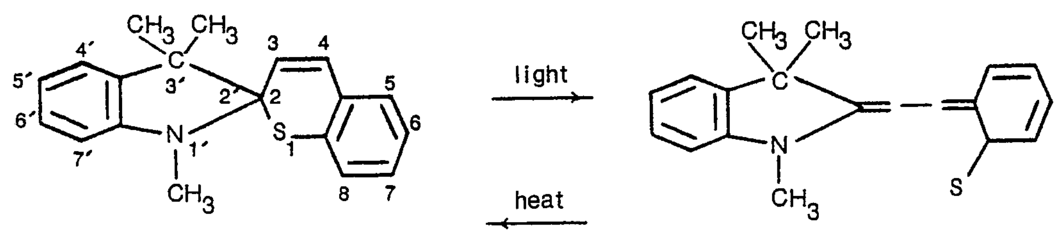


(Colourless) (Coloured)

Although this compound have a high absorption characteristic in the coloured state with respect to light having a wavelength ranging from 600 to 850 nm when placed in a 3-methylpentane solution at 77 K, it does not form any colour at normal temperatures, even in solution or in a polymer film. The compound is able to develop a colour only at temperatures lower than 0°C and the colour, once developed, disappears immediately after the compound returns to a normal temperature. Thus, this compound cannot in practice be used.

Chemical Abstracts, Vol. 98, 1983, pages 566 to 567, abstract no. 125243m, relates to an article published in Zh. Fiz. Khim. 1982, 56(11), 2794-8 (Russ) and dealing with the orbital nature of phosphorescent states of spiropyrans with a nitro group. The abstract discloses *inter alia* a compound having the formula

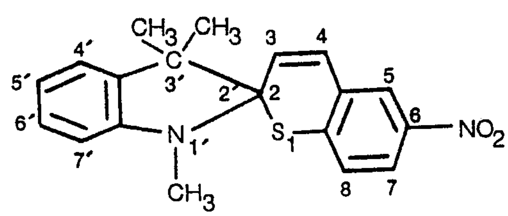

Photochromic compounds for use in photosensitive compositions embodying the invention and disclosed hereinbelow have a high absorption characteristic with respect to light having a wavelength higher than 700 nm, are stable in colour formation, are able to repeat colour formation and bleaching, depending on the temperature and ultraviolet (UV) irradiation, and can be used for recording by semiconductor laser.

The present invention provides a photosensitive composition comprising 97 to 50 wt% of a film-forming polymer and 3 to 50 wt% of a photochromic compound of the general formula

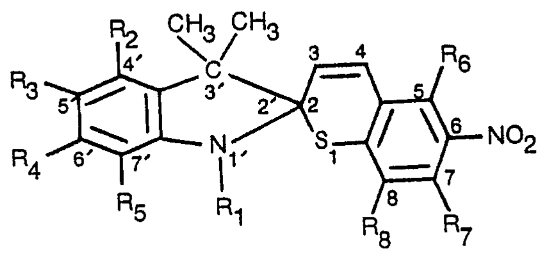

(I)

in which $R_1$ represents an alkyl group having 1 to 20 carbon atoms, each of $R_2$ and $R_4$ represents a hydrogen atom, $R_3$ represents a hydrogen or halogen atom, a nitro group, an alkyl or alkoxy group having 1 to 5 carbon atoms or a dimethylamino group, $R_5$ represents a hydrogen or halogen atom or an alkoxy group having 1 to 5 carbon atoms, $R_6$ represents a hydrogen or halogen atom, $R_7$ represents a hydrogen atom or an alkoxy group having 1 to 5 carbon atoms, and $R_8$ represents a hydrogen or halogen atom or an alkyl or alkoxy group having 1 to 5 carbon atoms.

Preferably, in formula (I) above, $R_1$ is an alkyl group having 1 to 10 carbon atoms, and each of $R_2$ and $R_4$ to $R_8$ represents a hydrogen atom. Such preferred compounds are represented by the following general formula

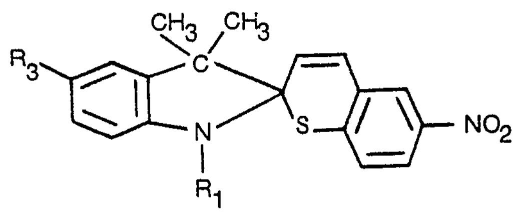

in which $R_1$ represents an alkyl group having 1 to 10 carbon atoms and $R_3$ represents a hydrogen atom, a halogen atom, an alkyl group having 1 to 5 carbon atoms, an alkoxy group having 1 to 5 carbon atoms, a nitro group, a cyano or dimethyl amino group.

Preferably, $R_3$ represents an alkoxy group having 1 to 5 carbon atoms or a dimethylamino group, and at least one of $R_5$, $R_7$ and $R_8$ represents an alkoxy group having 1 to 5 carbon atoms.

Specific examples of photochromic compounds for use in photosensitive compositions embodying the invention are indicated in the Examples and Table 1 appearing hereinbelow.

The photochromic compounds for use in the photosensitive composition of the present invention can be prepared according to the following reaction scheme (A)

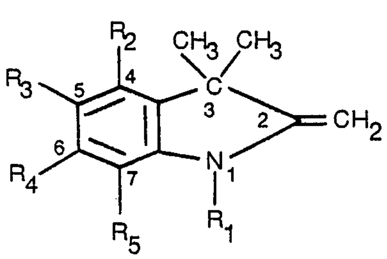

+

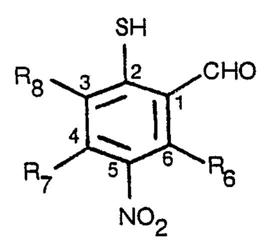

(II) (III)

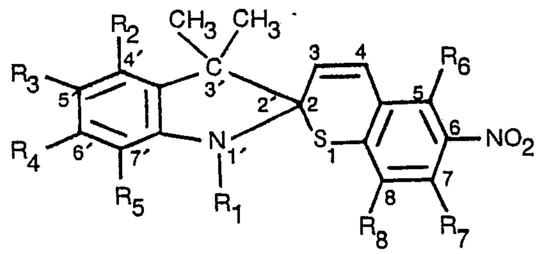

(A)

(I)

In other words, compounds having the formula (I) above can be prepared readily and in high yield by refluxing, in ethanol, a 1-alkyl-3,3-dimethyl-2-methyleneindoline (II) and a 5-nitro-thiosalicylaldehyde (III).

The starting compound (II) can be prepared according to the reaction scheme (B) given below, by alkylating 2,3,3-trimethylindolenine (IV) at the N position with an alkyl halide $R_1X$ in which X represents a halogen atom, thereby forming a salt (V), and subsequently treating the salt with an alkali.

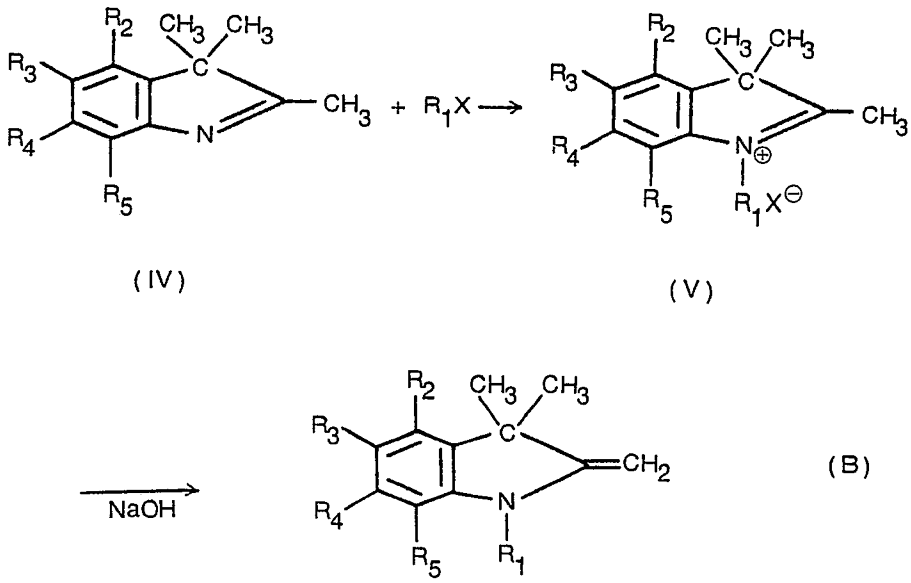


(IV)

(V)

(B)

(II)

For the alkylation at the N position, dialkylsulphates, alkyl toluenesulphonates and the like may also be used.

When $R_3$ in compound (II) is $(CH_3)_2N$- and each of $R_2$ and $R_4$ to $R_8$ is hydrogen, i.e. the compound (II) is 5-dimethylamino-1,3,3-trimethyl-2-methyleneindoline, it is necessary to use a specific technique for its preparation. This preparation process is believed first to have been devised by us. The compound is prepared according to the following reaction scheme (C) in which 5-amino-2,3,3-trimethylindolenine is reacted with methyl iodide in the presence of a hindered amine, e.g. 1,2,2,6,6-pentamethylpiperidine, to obtain a quaternary amine, and demethylating the quaternary amine by heating it with sodium n-propyl alcoholate. This is particularly described in Example 5 appearing hereinbelow.

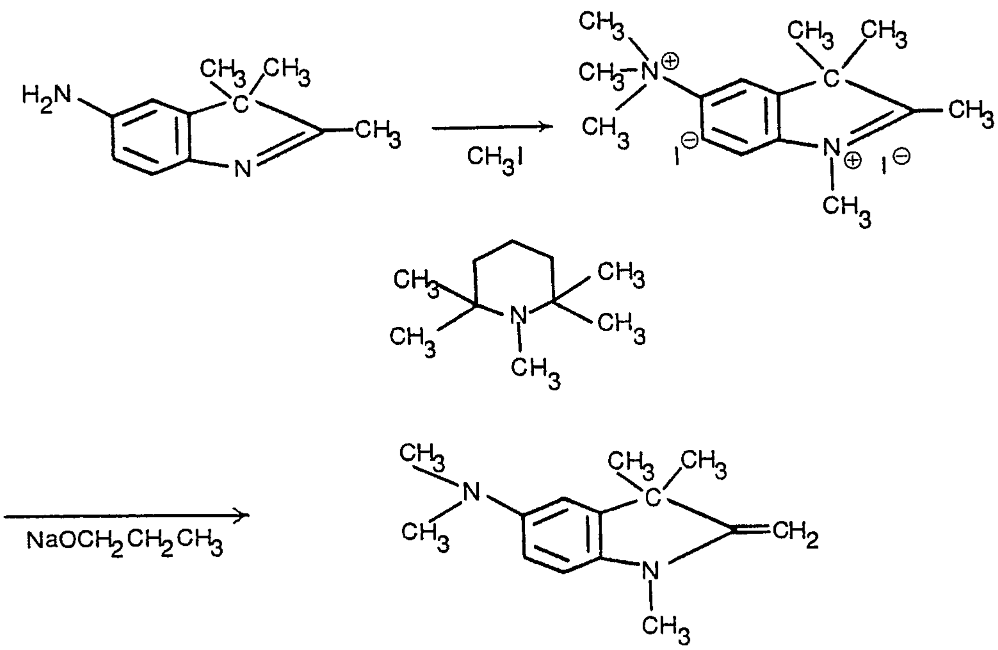


(C)

The compound (I) prepared according to the reaction scheme (A) using a compound (II) in which $R_3$ is a dimethylamino group has the feature that it is very stably coloured by UV irradiation.

The above-indicated 2,3,3-trimethylindolenine (IV) can be ordinarily obtained on the basis of a Fischer's indole synthesis, in which a substituted phenylhydrazine or hydrochloride thereof and 3-methyl-2-butanone are heated under acidic conditions. Alternatively, a substituted aniline and 3-methyl-3-bromo-2-butanone may be heated to obtain the compound (IV). Part of this preparation process is believed to be novel and is particularly described in Example 3 appearing hereinbelow.

Preparation of 5-nitrothiosalicylaldehyde (III) used in the reaction (A) is not known. This compound (III) can be prepared according to the reaction scheme (D) or (E) given below.

(1) Method of converting a halogen atom of an aldehyde compound carrying a halogen atom at the ortho position into an SH group (see Example 1):

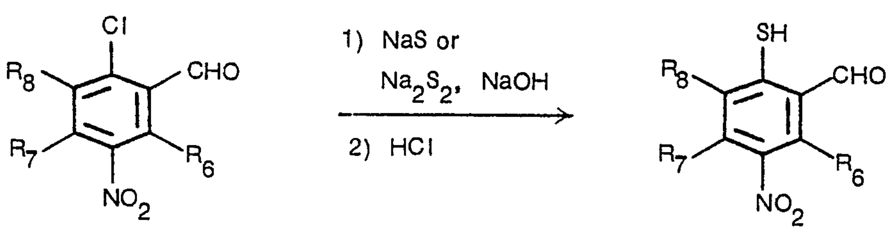


(D)

(VI) (III)

This reaction readily proceeds when $R_6$, $R_7$ and $R_8$ are all hydrogen atoms (such compound being readily available), but synthesis of substituted 5-nitro-2-mercaptobenzaldehydes is very difficult. In this case, a method using the following reaction scheme (E) is used.

(2) Method of converting the OH group of salicylaldehyde into an SH group:

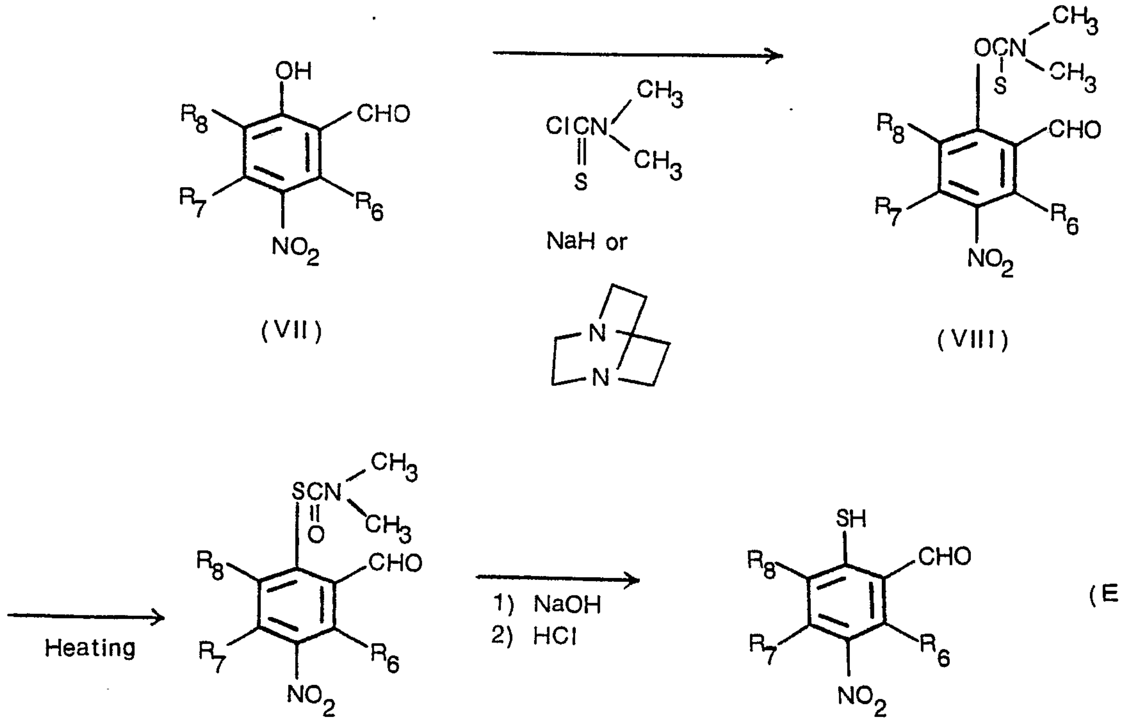


(VII) (VIII)

(IX) (III) (E)

Dimethylcarbamate (VIII) is prepared from the salicylaldehyde (VII), followed by heating to cause a rearrangement reaction of O and S to obtain a compound (IX) and subsequent alkaline hydrolysis to give 2-mercaptobenzaldehyde (III). The reactions of this series are known *per se*. However it is believed not known to apply these reactions so as to obtain a compound of formula (III). The reaction scheme (E) is advantageous in that synthesis of the starting salicylaldehyde is easy and yields at the respective reaction stages are high. Accordingly, for the synthesis of 5-nitrosalicylaldehyde having substituents, use of a process according to the reaction scheme (E) is favourable. In this process, the reaction can be caused to proceed so that compound (VII) can be converted into compound (IX) in one step, as will be seen in Example 4 appearing hereinbelow.

The photosensitive compositions or materials embodying the present invention, which have once been coloured, may be bleached or returned to their original colourless state by heating to 60 to 80°C or by irradiation by visible light having a wavelength higher than 500 nm. UV irradiation enables the material to be coloured. Thus, the material can stand repeated use.

These compounds have very wide utility in various fields. For instance, they can be used as a medium for semiconductor laser recording and reproducing purposes and also as various recording materials, such as memory materials, duplication materials, photosensitive materials for printing, recording materials for cathode ray tubes, and photosensitive materials for holography. Also, the materials can be utilised as materials for optical filters, display, masking, actionometers, decoration and the like.

Photochromic compounds for use in photosensitive compositions embodying the invention are soluble in various solvents, including alcohols such as methanol, ethanol and isopropyl alcohol, ketones such as acetone, methyl ethyl ketone and cyclohexanone, ethers such as ethyl ether, dioxane and tetrahydrofuran, esters such as ethyl acetate and n-butyl acetate, benzene, toluene, xylene, n-hexane, cyclohexane, acetonitrile, dimethylformamide, dimethylsulphoxide, chloroform, and mixtures thereof. When the compound is to be applied as a photochromic material or medium, it can be dissolved in a solvent along with a polymer material. The resulting solution can be used for film formation or applied to a support and dried. Alternatively, the compound may be kneaded along with a polymer material without the use of any solvent and subjected to film formation.

The polymer materials useful in the practice of the invention may be any materials which are miscible with the compounds of the general formula (I) and which have excellent film-forming ability. Examples of such polymers include polymethyl methacrylate, polystyrene, polyvinyl acetate, polyvinylbutyral, cellulose acetate, polyvinyl chloride, polyvinylidene chloride, vinyl chloride-vinyl acetate copolymer, polypropylene, polyethylene, polyacrylonitrile, urethane resins, epoxy resins, polyesters, phenolic resins and phenoxy resins. Of these, chlorine-containing polymers are preferable because of higher stability after colour formation. The amount of the compound represented by the general formula (I) in a photosensitive composition comprising a polymer material is in the range of from 3 to 50 wt%, preferably from 5 to 30 wt%. Amounts smaller than 3 wt% are unfavourable because this gives lower optical density. On the other

hand, amounts larger than 50 wt% result in precipitation of the compound (I) from the polymer material.

The present invention will now be particularly described by way of the following examples, which are illustrative of the photochromic compounds that can be used in the compositions of the invention.

Example 1

Preparation of 1′,3′,3′-trimethyl-6-nitrospiro-[2H-1-benzothiopyran-2,2′-indoline]:

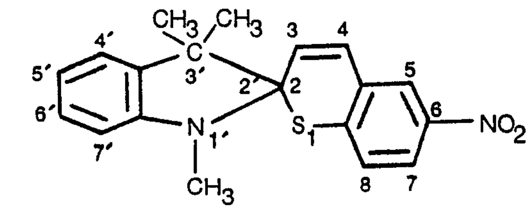

5-nitro-2-mercaptobenzaldehyde,

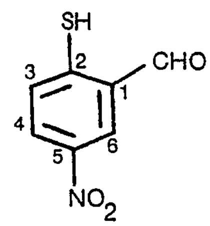

which is one of starting materials, was prepared according to the following reaction scheme

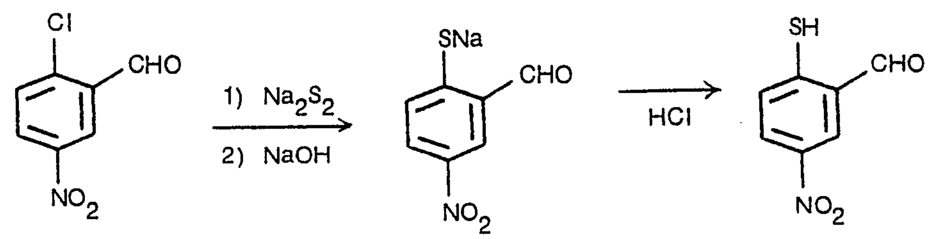


5 g of 2-chloro-5-nitrobenzaldehyde was added to 10 cc of ethanol, followed by heating under refluxing conditions. Thereafter, a mixture of 4.66 g of $Na_2S.9H_2O$ and 0.62 g of S was heated to obtain $Na_2S_2$. The thus-obtained $Na_2S_2$ was added to a boiling solution of 2-chloro-5-nitrobenzaldehyde in ethanol over 15 minutes. After completion of the addition, 10 ml of a solution containing 1.08 g of NaOH in 95% ethanol was further added over 30 minutes. After completion of the addition, the mixture was cooled and poured into iced water consisting of 30 g of ice and 400 cc of water, after which insoluble matter was filtered off. The resulting filtrate was neutralised with HCl, thereby obtaining a yellow precipitate. The precipitate was collected by filtration. Since the yellow precipitate contained impurities, it was dissolved by heating it in 20 ml of ethanol, to which was added a solution containing 1.08 g of NaOH in 95% ethanol, followed by removing insoluble matter by filtration. The filtrate was again neutralised with HCl and cooled to obtain yellow crystals of the intended 5-nitro-2-mercaptobenzaldehyde. The crystals were collected by filtration and dried for use in a subsequent reaction. The yield was 3.15 g (64%) and the melting point was 85 to 88°C.

Thereafter, 1.6 g of commercially available 1,3,3-trimethyl-2-methyleneindoline and 2 g of the 5-nitro-2-mercaptobenzaldehyde obtained in the manner described above were placed in 100 ml of methanol and heated under refluxing conditions for 2 hours. The solution was concentrated and methanol was added thereto, thereby precipitating yellow crystals. The crystals were recrystallised from benzene-methanol to obtain the intended 1′,3′,3′-trimethyl-6-nitrospiro[2H-1-benzothiopyran-2,2′-indoline]. The yield was found to be 1.1 g (35%) and the melting point was 178 to 179°C.

The 5-nitro-2-mercaptobenzaldehyde prepared as the starting material was analysed by proton-NMR spectroscopy. TMS (tetramethylsilane) was used as an internal standard and $CDCl_3$ (chloroform-$d_1$) was used as a solvent. The measurement was effected by the use of a JNM-FX60Q NMR apparatus made by Nippon Electronics Co., Ltd. The results are shown below with the compound being identified.

Chemical Shift (δ, ppm)

6.16 (singlet, SH, 1H)

7.48 (doublet, H at 3 position, 1H)

8.22 (double doublet, H at 4 position, 1H)
8.61 (doublet, H at 6 position, 1H)
10.10 (singlet, CH0, 1H)

Likewise, the 1′,3′,3′-trimethyl-6-nitrospiro-[2H-1-benzothiopyran-2,2′-indoline] was subjected to spectroscopic measurement in the same manner as described above and identified by the following proton-NMR spectrum data.

δ (ppm)
1.24 (singlet, C—$CH_3$, 3H)
1.40 (singlet, C—$CH_3$, 3H)
2.64 (singlet, N—$CH_3$, 3H)
5.96 (doublet, H at 3 position, 1H)
6.44 (doublet, H at 7′ position, 1H)
6.6—7.4 (multiplet, aromatic H, H at 4 position, 5H)
7.7—8.1 (multiplet, H at 5,7 positions, 2H)

A solution of 3 parts by weight of the thus-obtained 1′,3′,3′-trimethyl-6-nitrospiro[2H-1-benzothiopyran-2,2′-indoline] and 10 parts by weight of vinyl chloride-vinylidene chloride copolymer (Denka Vinyl No. 1000W, supplied by Denki Chem. Ind. Co., Ltd) as a polymer material, in 100 parts by weight of a solvent (tetrahydrofuran : cyclohexanone = 1:1 (by volume)), was prepared. The solution was spin-coated onto a quartz substrate and dried at 80°C for 2 hours to obtain a sample having a photosensitive layer 1.5 µm thick.

The thus-obtained sample was exposed to ultraviolet light, having a wavelength of about 360 nm, from a 500 W super high pressure mercury lamp (Ushio Electrical Machinery Co., Ltd) through a glass filter (UV-D360, supplied by Toshiba Corp.). The light-yellow sample was changed to dark-green in colour. The absorption spectra were measured by a self-recording spectrophotometer Model 320 made by Hitachi, Ltd. It was found that the sample which had been irradiated with UV light had a maximum absorbance at 680 nm and the absorption extended to about 900 nm. The maximum absorbance at the wavelength of 680 nm was about 0.6. The same sample had a significant absorption at 780 nm and the rate of the absorbance based on the absorbance at the absorption maximum wavelength ($\lambda_{max}$) was 47%.

For comparison, the above procedure was repeated using 1′,3′,3′-trimethyl-6-nitrospiro [2H-1-benzopyran-2-2′-indoline], which is also a known spiropyran compound. The colour formation characteristic of the resulting photosensitive film was determined. It was found that $\lambda_{max}$ was 580 nm and the end of absorption at the long wavelength side took place at 700 nm.

Example 2

Preparation of 5′-methoxy-1′-n-hexyl-3′,3′-dimethyl-6-nitro-8-methoxyspiro [2H-1-benzothiopyran-2,2′-indoline]:

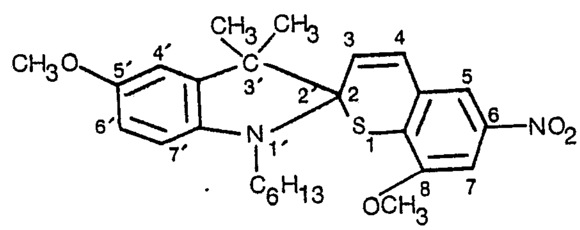

3-methoxy-5-nitro-2-mercaptobenzaldehyde,

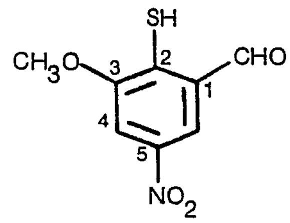

which was one of starting materials, was prepared in the following manner. 10 g of 3-methoxy-5-nitrosalicylaldehyde and 11.4 g of 1,4-diazabicyclo [2,2,2] octane were dissolved in DMF (dimethylformamide). To the solution was added a solution of 9.4 g of dimethylthiocarbamoyl chloride in DMF, after which reacting took place at 50 to 60°C for 1.5 hours. Thereafter, 300 ml of water was added to

the reaction solution and the resulting precipitate was filtered, and recrystallised from methanol to obtain 11.1 g (yield 77%) of dimethylthiocarbamate

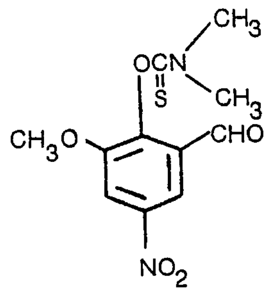

This dimethylthiocarbamate was heated on an oil bath at 160°C and melted, after which it was cooled and admixed with methanol. The resulting crystals were filtered to obtain 10.3 g (yield 93%) of a compound of the following formula

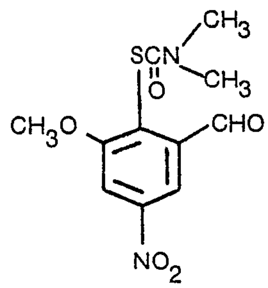

The compound was dissolved in 300 ml of methanol, to which was added 30 ml of an aqueous 4N NaOH solution at a normal temperature, which was followed by reaction for 1 hour while blowing $N_2$ gas into the reaction system. Concentrated hydrochloric acid was subsequently added to the reaction system to render the system acidic, which was followed by refluxing for 40 minutes, cooling, adding 100 cc of water, and filtering the resulting crystals, thereby obtaining 7.65 g (yield 99%) of 3-methoxy-5-nitro 2-mercaptobenzaldehyde. The melting point was 169 to 170°C. The compound was subjected to proton-NMR spectroscopy in the same manner as in Example 1 with the results shown below. From the above results, the compound was identified as being that intended.

δ (ppm)
4.09 (singlet, $OCH_3$, 3H)
6.10 (singlet, SH, 1H)
7.85 (doublet, H at 4 position, 1H)
8.31 (doublet, H at 6 position, 1H)
10.41 (singlet, CHO, 1H)

Subsequently, 4.6 g of the 2-mercaptobenzaldehyde and 5.9 g of 1-n-hexyl-3,3-dimethyl-5-methoxy-2-methyleneindoline (which was obtained by reacting 5-methoxy-2,3,3-trimethylindolenine and hexyl bromide in chloroform and treating the resulting produce with NaOH)

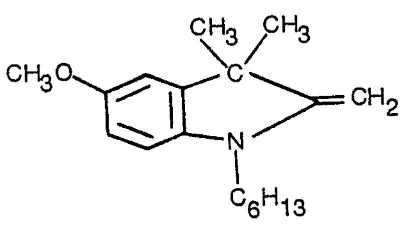

were heated and refluxed in 100 ml of ethanol for 2 hours. The solution was concentrated and subjected to isolation by silica gel column chromatography, followed by recrystallisation with benzene-methanol to obtain 4.6 g of the intended 5′-methoxy-1′-n-hexyl-3′,3′-dimethyl-6-nitro-8-methoxyspiro [2H-1-benzothiopyran-2,2′-indoline]. The yield was 45% and the melting point was 97 to 98°C.

$^1$H-NMR Spectrum Data
δ (ppm)
0.88 (triplet, n-hexyl group $CH_3$, 3H)
1.23 (singlet, C—$CH_3$, 3H)

1.36 (singlet, C—$CH_3$, 3H)
1.0—2.0 (multiplet, n-hexyl group $CH_2$, 8H)
3.04 (triplet, N—$CH_2$, 2H)
3.77 (singlet, $OCH_3$, 3H)
3.90 (singlet, $OCH_3$, 3H)
5.94 (doublet, H at 3 position, 1H)
6.40 (doublet, H at 7′ position, 1H)
6.5—6.8 (multiplet, H at 4′,6′ positions, 2H)
6.84 (doublet, H at 4 position, 1H)
7.52 (doublet, H at 7 position, 1H)
7.74 (doublet, H at 5 position, 1H)

The compound of this example was dissolved in DMF and degassed. Upon irradiation by ultraviolet light at about 360 nm, the solution was coloured green. When the coloured solution was heated, the solution was bleached. The colouring and bleaching cycle could be repeated many times.

Example 3

Preparation of 5′,7′-dimethoxy-1′,3′,3′-trimethyl-6-nitro-8-methoxyspiro [2H-1-benzothiopyran-2,2′-indoline]:

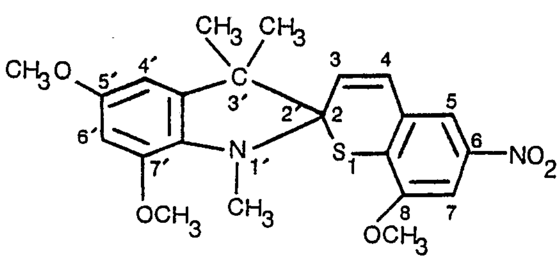

5,7-dimethoxy-1,3,3-trimethyl-2-methyleneindoline,

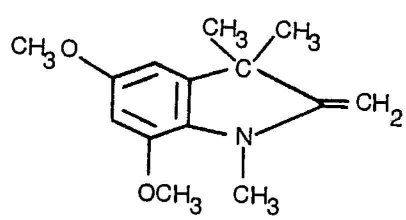

which was one of starting materials, was prepared as follows. 20 g of 3-bromo-3-methyl-2-butanone and 55.5 g of 2,4-dimethoxyaniline were heated at 140°C for 1 hour, and then extracted with chloroform. The chloroform phase was treated with a diluted hydrochloric acid solution comprising 18 ml of concentrated hydrochloric acid diluted with 200 ml of water. An excess of the dimethoxyaniline was removed from the solution, which was then distilled under reduced pressure to obtain 16.4 g of 5,7-dimethoxy-2,3,3-trimethylindolenine (yield 62%, boiling point 107°C at 27 Pa (0.2 mmHg).

5 g of the indolenine and 4 g of methyl iodide were dissolved in 5 ml of methanol. The solution was hermetically sealed and reacted at 110°C for 2 hours. The resulting powder produce was washed with diethyl ether, dried and treated with NaOH to obtain 3.7 g (yield 70%) of 5,7-dimethoxy-1,3,3-trimethyl-2-methyleneindoline.

3.4 g of the indoline and 3.2 g of the 3-methoxy-5-nitrosalicylaldehyde obtained in Example 2 were reacted in the same manner as in Example 2 to obtain 4.3 g of the intended 5′7′-dimethoxy-1′,3′,3′-trimethyl-6-nitro-8-methoxyspiro [2H-1-benzothiopyran-2,2′-indoline] (yield 69%, melting point 131°C).

The compound was identified by proton-NMR spectroscopy. A DMF solution of the compound exhibited a colour and bleaching phenomenon similar to the compound of Example 2.

Example 4

Preparation of 5'-methoxy-1',3',3'-trimethyl-6-nitro-8-chlorospiro [2H-1-benzothiopyran-2,2'-indoline]:

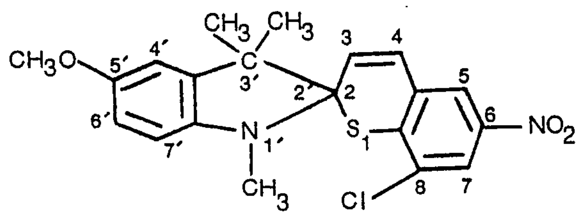

3-chloro-5-nitro-2-mercaptobenzaldehyde,

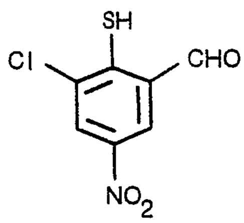

which was one of starting materials, was prepared as follows. 10 g of 3-chloro-5-nitrosalicylaldehyde was dissolved in 300 ml of DMF, to which was added 1.2 g of NaH under ice-cooling conditions. When generation of hydrogen stopped, a solution of 8 g of dimethylthiocarbamoyl chloride in 10 ml of DMF was added, followed by reaction at 40°C for 15 minutes and then at 80°C for 1 hour. After cooling, iced water was added to the reaction mixture and the resulting crystals were filtered and recrystallised from benzene-ethanol. 7.3 g (yield 51%) of a compound of the following formula was obtained.

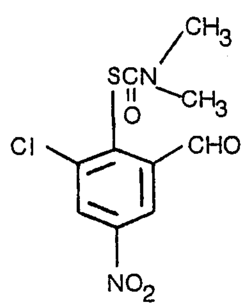

5 g of the compound was dissolved in 25 ml of ethanol, to which was added 23.5 ml of 4N NaOH aqueous solution, which was followed by reaction at ambient temperature for 2 hours while blowing $N_2$ gas. The reaction mixture was rendered acidic by the use of dilute hydrochloric acid and the resulting yellow crystals were filtered and dried to obtain 3.72 g of 3-chloro-5-nitrothio-salicylaldehyde (yield 99%, melting point 84 to 89°C).

2.5 g of the salicylaldehyde and 2.5 g of 5-methoxy-1,3,3-trimethyl-2-methyleneindoline were reacted in the same manner as in Example 2 and the resulting product was separated to obtain 3.3 g of the intended 5'-methoxy-1',3',3'-trimethyl-6-nitro-8-chlorospiro[2H-1-benzothiopyran-2,2'-indoline] (yield 67%). The melting point was 168 to 169°C. The compound was identified by proton-NMR spectroscopy.

4 parts by weight of the thiopyran-type spiropyran compound obtained in this example and 100 parts by weight of vinyl chloride-vinylidene chloride copolymer (Denka Vinyl No. 1000W, supplied by Denki Chem. Ind. Co., Ltd) were dissolved in 100 parts by weight of a mixture of tetrahydrofuran and cyclohexanone in a mixing ratio by volume of 1:1. The solution was spin-coated onto a quartz glass plate and dried *in vacuo* at 80°C for 2 hours to obtain a sample having a 1 μm thick photosensitive film. The sample was irradiated by ultraviolet light of about 360 nm from a 500 W super high pressure mercury lamp (made by Ushio Electric Co., Ltd) through a UV-360 filter (made by Toshiba Corp.), whereupon the sample developed a green colour. The coloured sample had an absorption maximum wavelength of 650 nm, at which a saturation absorbance was 0.6. The absorbance was found to be 0.43 at 720 nm and 0.14 at 780 nm. The coloured sample was bleached when heated to 80°C and was again coloured by irradiation by ultraviolet light. This phenomenon could be repeated many times.

Example 5

Preparation of 5′-dimethylamino-1′,3′,3′-trimethyl-6-nitrospiro[2H-1-benzothiopyran-2,2′-indoline]:

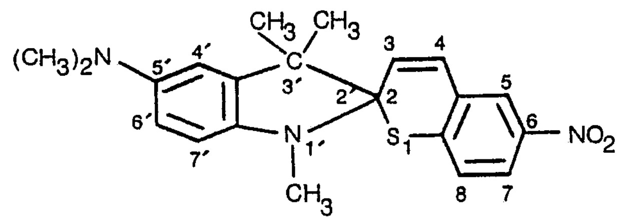

5-dimethylamino-1,3,3-trimethyl-2-methyleneindoline

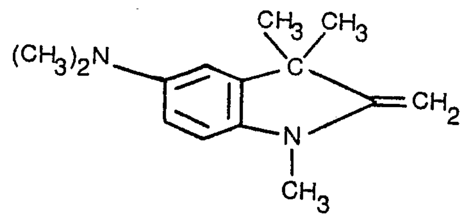

was prepared as follows. 3.74 g of 5-amino-2,3,3-trimethylindolenine, 6.61 g of 1,2,2,6,6-pentamethylpiperidine and 18.27 g of methyl iodide were dissolved in 40 ml of DMF. The solution was allowed to stand at room temperature for 15 hours. The resulting crystals were washed with an acetone solution containing 6% DMF and then with acetone, which was followed by drying and the addition of 150 ml of n-propyl alcohol containing 23.5 g of sodium. The mixture was refluxed for demethylation over 17 hours and the n-propyl alcohol was removed under reduced pressure. Water was added to the reaction solution, which was extracted with ether to obtain 4.21 g of 5-dimethylamino-1,3,3-trimethyl-2-methyleneindoline (yield 91%).

3 g of the thus-obtained indoline and 3 g of the 5-nitrothiosalicylaldehyde obtained in Example 1 were heated in 40 ml of ethanol for 4 hours, followed by treatment in the same manner as in Example 2 to obtain 2.4 g of the intended 5′-dimethylamino-1′,3′,3′-trimethyl-6-nitrospiro[2H-1-benzothiopyran-2,2′-indoline] (yield 45%, melting point 166.5°C to 167°C). For identification, proton-NMR spectroscopy was used.

The compound of this example was used to make a polymer film in the same manner as in Example 4. When the film sample was irradiated by UV light, a blackish purple colour developed. The absorption spectra were as follows: the absorption maximum wavelength was 620 nm and the saturation absorbance at that wavelength (when the sample was irradiated by UV light and the absorbance of the coloured sample reached saturation) was 0.62. The absorbance was 0.42 at 720 nm and 0.13 at 780 nm. When the coloured photosensitive film sample was heated to 80°C, absorption of light having a wavelength over 700 nm disappeared. This absorption reappeared upon irradiation by UV light. The colour formation of the sample was very stable.

Thiopyran-type spiropyran compounds having various substituents as indicated in Table 1 were prepared in the same manner as in Examples 1 to 5. The compounds were identified by proton-NMR spectroscopy. The compounds were used to make photosensitive films in the same manner as in Example 4. The films were irradiated to such an extent that colour formation reached saturation. The results are shown in Table 1. Absorbances at certain wavelengths were absorbance values of 1 micron thick photosensitive films. From the results, it will be seen that the compounds embodying the present invention have high absorptions at wavelengths over 700 nm.

As will be understood from the foregoing, thiopyran-base spiropyran photochromic compounds embodying the present invention, in which the oxygen atom in the benzopyran structure of a known spiropyran compound is replaced by a sulphur atom and which have an $NO_2$ group at the 6 position, become sensitive to light of a wavelength longer by about 100 nm than known spiropyran compounds and have high absorption characteristics with respect to light having a wavelength of over 700 nm.

TABLE 1

| Substituent | | | | | | | | Melting point (°C) | Absorption maximum wavelength $\lambda_{max}$ (nm) | Absorbance | | |
|---|---|---|---|---|---|---|---|---|---|---|---|---|
| $R_1$ | $R_2$ | $R_3$ | $R_4$ | $R_5$ | $R_6$ | $R_7$ | $R_8$ | | | $\lambda_{max}$ | 720 nm | 780 nm |
| CH3 | H | H | H | H | H | H | H | 178—179 | 680 | 0.48 | 0.43 | 0.22 |
| $CH_3$ | H | $NO_2$ | H | H | H | H | H | 221—223 | 750 | 0.24 | 0.23 | 0.22 |
| $CH_3$ | H | Cl | H | H | H | H | H | 139—139.5 | 690 | 0.53 | 0.51 | 0.33 |
| $CH_3$ | H | $CH_3$ | H | H | H | H | H | 137—138 | 670 | 0.44 | 0.37 | 0.19 |
| $CH_3$ | H | $OCH_3$ | H | H | H | H | H | 160—162 | 660 | 0.57 | 0.47 | 0.19 |
| $CH_3$ | H | $OCH_3$ | H | H | H | H | $OCH_3$ | 165—166 | 690 | 0.45 | 0.42 | 0.25 |
| $C_6H_{13}$ | H | $OCH_3$ | H | H | H | H | $OCH_3$ | 97—98 | 690 | 0.56 | 0.52 | 0.29 |
| $CH_3$ | H | $CH_3$ | H | H | H | H | $OCH_3$ | 224—225 | 695 | 0.53 | 0.51 | 0.33 |
| $CH_3$ | H | H | H | H | H | H | $OCH_3$ | 180—181 | 700 | 0.55 | 0.54 | 0.37 |
| $CH_3$ | H | Cl | H | H | H | H | $OCH_3$ | 239—240 | 720 | 0.53 | 0.53 | 0.43 |
| $CH_3$ | H | $NO_2$ | H | H | H | H | $OCH_3$ | 242 | 770 | 0.18 | 0.16 | 0.18 |
| $CH_3$ | H | $OCH_3$ | H | $OCH_3$ | H | H | $OCH_3$ | 131 | 675 | 0.34 | 0.28 | 0.15 |
| $C_6H_{13}$ | H | $OCH_3$ | H | $OCH_3$ | H | H | $OCH_3$ | 93 | 675 | 0.55 | 0.45 | 0.20 |
| $CH_3$ | H | $OCH_3$ | H | H | H | H | Cl | 168—169 | 650 | 0.60 | 0.43 | 0.14 |
| $CH_3$ | H | $OCH_3$ | H | H | H | H | Br | 193—194 | 645 | 0.64 | 0.42 | 0.14 |
| $CH_3$ | H | $OCH_3$ | H | H | H | H | $CH_3$ | 177—178 | 675 | 0.52 | 0.47 | 0.22 |
| $CH_3$ | H | $OCH_3$ | H | H | H | $OCH_3$ | H | 193—194 | 650 | 0.59 | 0.38 | 0.09 |
| $C_6H_{13}$ | H | $OCH_3$ | H | H | Cl | H | Cl | 96 | 610 | 0.57 | 0.13 | 0.03 |

TABLE 1

| Substituent | | | | | | | | Melting point (°C) | Absorption maximum wavelength $\lambda_{max}$ (nm) | Absorbance | | |
|---|---|---|---|---|---|---|---|---|---|---|---|---|
| $R_1$ | $R_2$ | $R_3$ | $R_4$ | $R_5$ | $R_6$ | $R_7$ | $R_8$ | | | $\lambda_{max}$ | 720 nm | 780 nm |
| $C_6H_{13}$ | H | $OCH_3$ | H | H | H | H | Cl | 110 | 650 | 0.70 | 0.45 | 0.14 |
| $CH_3$ | H | $N(CH_3)_2$ | H | H | H | H | H | 167 | 620 | 0.62 | 0.42 | 0.13 |
| $CH_3$ | H | $N(CH_3)_2$ | H | H | H | H | $CH_3$ | 156 | 640 | 0.31 | 0.36 | 0.13 |
| $CH_3$ | H | $N(CH_3)_2$ | H | H | H | H | $OCH_3$ | 187 | 670 | 0.30 | 0.24 | 0.12 |
| $CH_3$ | H | $N(CH_3)_2$ | H | Cl | H | H | H | 170—172 | 670 | 0.37 | 0.30 | 0.15 |
| $CH_3$ | H | $N(CH_3)_2$ | H | Cl | H | H | $OCH_3$ | 204—206 | 710 | 0.16 | 0.16 | 0.11 |
| $C_6H_{13}$ | H | $OCH_3$ | H | H | H | H | H | Oily, viscous matter | 665 | 0.50 | 0.40 | 0.16 |

## Claims

1. A photosensitive composition comprising 97 to 50 wt% of a film-forming polymer and 3 to 50 wt% of a photochromic compound of the following general formula

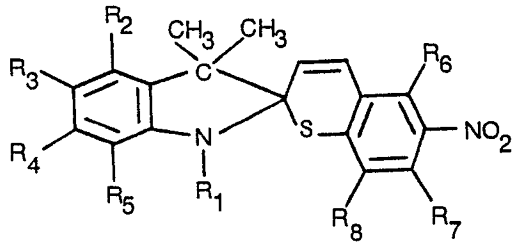

in which $R_1$ represents an alkyl group having 1 to 20 carbon atoms, each of $R_2$ and $R_4$ represents a hydrogen atom, $R_3$ represents a hydrogen or halogen atom, a nitro group, an alkyl or alkoxy group having 1 to 5 carbon atoms or a dimethylamino group, $R_5$ represents a hydrogen or halogen atom or an alkoxy group having 1 to 5 carbon atoms, $R_6$ represents a hydrogen or halogen atom, $R_7$ represents a hydrogen atom or an alkoxy group having 1 to 5 carbon atoms, and $R_8$ represents a hydrogen or halogen atom or an alkyl or alkoxy group having 1 to 5 carbon atoms.

2. A photosensitive composition according to claim 1, wherein $R_3$ represents an alkoxy group having 1 to 5 carbon atoms or a dimethylamino group, and at least one of $R_5$, $R_7$ and $R_8$ represents an alkoxy group having 1 to 5 carbon atoms.

3. A photosensitive composition according to claim 1 or claim 2, wherein the polymer is a chlorine-containing polymer.

4. A photosensitive material comprising a film of the composition according to claim 1, claim 2 or claim 3.

## Patentansprüche

1. Photoempfindliche Masse, enthaltend 97 bis 50 Gew.-% eines filmbildenden Polymeren und 3 bis 50 Gew.-% einer photochromen Verbindung der folgenden allgemeinen Formel

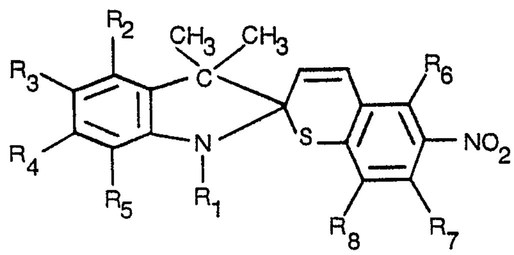

in der $R_1$ eine Alkylgruppe mit 1 bis 20 Kohlenstoffatomen, jeder Rest $R_2$ und $R_4$ ein Wasserstoffatom, $R_3$ ein Wasserstoff- oder Halogenatom, eine Nitrogruppe, eine Alkyl- oder Alkoxygruppe mit 1 bis 5 Kohlenstoffatomen oder eine Dimethylaminogruppe, $R_5$ ein Wasserstoff- oder Halogenatom oder eine Alkoxygruppe mit 1 bis 5 Kohlenstoffatomen, $R_6$ ein Wasserstoff- oder Halogenatom, $R_7$ ein Wasserstoffatom oder eine Alkoxygruppe mit 1 bis 5 Kohlenstoffatomen und $R_8$ ein Wasserstoff- oder Halogenatom oder eine Alkyl- oder Alkoxygruppe mit 1 bis 5 Kohlenstoffatomen darstellen.

2. Photoempfindliche Masse gemäß Anspruch 1, wobei $R_3$ eine Alkoxygruppe mit 1 bis 5 Kohlenstoffatomen oder eine Dimethylaminogruppe darstellt und wenigstens einer der Reste $R_5$, $R_7$ und $R_8$ eine Alkoxygruppe mit 1 bis 5 Kohlenstoffatomen ist.

3. Photoempfindliche Masse gemäß Anspruch 1 oder 2, in der das Polymere ein Chlor enthaltendes Polymer ist.

4. Photoempfindliches Material, bestehend aus einem Film aus der Masse gemäß den Ansprüchen 1, 2 oder 3.

**Revendications**

1. Une composition photosensible constituée de 97 à 50% en poids d'un polymère filmogène et de 3 à 50% en poids d'un composé photochrome répondant à la formule générale suivante

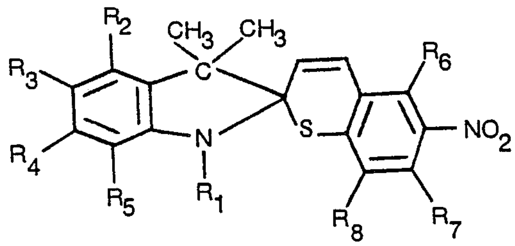

dans laquelle $R_1$ représente un groupe alkyle en $C_1$—$C_{20}$, chacun des symboles $R_2$ et $R_4$ représente un atome d'hydrogène, $R_3$ représente un atome d'hydrogène ou d'halogène, un groupe nitro, un groupe alkyle ou alcoxy en $C_1$—$C_5$ ou un groupe diméthylamino, $R_5$ représente un atome d'hydrogène ou d'halogène ou un groupe alcoxy en $C_1$—$C_5$, $R_6$ représente un atome d'hydrogène ou d'halogène, $R_7$ représente un atome d'hydrogène ou un groupe alcoxy en $C_1$—$C_5$ et $R_8$ représente un atome d'hydrogène ou d'halogène ou un groupe alkyle ou alcoxy en $C_1$—$C_5$.

2. Une composition photosensible selon la revendication 1, dans laquelle $R_3$ représente un groupe alcoxy en $C_1$—$C_5$ ou un groupe diméthylamino et l'un au moins des symboles $R_5$, $R_7$ et $R_8$ représente un groupe alcoxy en $C_1$—$C_5$.

3. Une composition photosensible selon la revendication 1 ou 2, dans laquelle le polymère est un polymère contenant du chlore.

4. Une matière photosensible comprenant une pellicule à la composition selon la revendication 1, 2 ou 3.